# EUROPÄISCHE PATENTSCHRIFT

(11) **EP 2 381 933 B1**
(45) Veröffentlichungstag und Bekanntmachung des Hinweises auf die Patenterteilung: **25.06.2014**
(21) Anmeldenummer: 09795937.3
(22) Anmeldetag: 22.12.2009
(51) Int. Cl.: B26D 3/08, A61K 9/70, A61F 13/02, B26F 1/20, B26F 1/38, B26F 3/10, B26D 7/10

(54) **PFLASTER-HERSTELLUNGSTECHNOLOGIE**
PATCH PRODUCTION TECHNOLOGY
TECHNOLOGIE DE FABRICATION D'UN PANSEMENT

(30) Priorität: 29.12.2008 EP 08173017
(43) Veröffentlichungstag der Anmeldung: 02.11.2011
(73) Patentinhaber: UCB Pharma GmbH, 40789 Monheim (DE)
(72) Erfinder: JASCH, Ingolf, 42659 Solingen (DE)
(74) Vertreter: UCB Intellectual Property
(86) Internationale Anmeldenummer: PCT/EP2009/009207
(87) Internationale Veröffentlichungsnummer: WO 2010/075992

(56) Entgegenhaltungen:
- EP-A- 0 013 606
- EP-A- 1 325 742
- WO-A-03/092677
- WO-A-2007/147556
- WO-A-2008/048829
- WO-A-2009/009651
- DE-C1- 3 430 250
- FR-A- 929 456
- US-A- 4 573 996
- US-A- 5 405 486

## Beschreibung

### Technisches Gebiet

Die Erfindung betrifft ein transdermales Pflaster mit einer Release-Liner-Folie, einer Wirkstoffschicht mit dem Wirkstoff Rotigotin oder einem seiner pharmakologisch akzeptablen Salze und einer Trägerschicht, wobei die Wirkstoffschicht zwischen der Release-Liner-Folie und der Trägerschicht eingebracht ist. Des Weiteren betrifft die Erfindung ein Verfahren zur Herstellung eines transdermalen Pflasters.

### Hintergrund

Transdermale Pflaster dienen der Applikation von Arzneistoffen durch die Haut. Nach Aufkleben eines transdermalen Pflasters auf die Haut gelangt ein für diese Anwendung geeigneter Arzneistoff kontinuierlich direkt in den systemischen Blutkreislauf, d.h. unter Umgehung des Magen -Darm- Traktes und der ersten Leberpassage , wodurch z.B. Magenunverträglichkeit und/oder der frühzeitige hepatische "first-pass" Effekt (Abbau in der Leber) bestimmter Substanzen nach oraler Gabe vermieden wird. Beispiele hierfür sind Nikotinpflaster, Hormonpflaster und Schmerzpflaster sowie transdermale Pflaster zur Behandlung der Parkinson-Krankheit oder für die Behandlung der ruhelosen Beine (restless legs). Solche therapeutischen Pflaster können beispielsweise den dopaminergen Wirkstoff Rotigotin enthalten. Er ist unter anderem zur Behandlung der Parkinsonschen Krankheit und zur Therapie der ruhelosen Beine ("restless legs") geeignet und entsprechende transdermale Pflaster werden bereits in einigen Ländern angewendet. Medizinische Verwendungen von Rotigotin bzw. Rotigotin-haltigen Arzneiformen sind beispielsweise beschrieben in WO 2005/92331, WO 2005/009424, WO 2007/147556, WO 03/92677 sowie WO 2005/63237.

Eine aus dem Stand der Technik bekannte und bevorzugte Ausführungsform eines transdermalen Pflasters besteht aus einer Wirkstoffschicht, einer Trägerschicht und einer Release Liner Folie. So aufgebaute Pflaster werden üblicherweise aus einem großflächigen Laminat ausgestanzt.

Nach dem Stanzen ist die Wirkstoffschicht an den umfangseitigen Trennkanten offen, d.h. nicht durch Folien abgedeckt.

Eine mögliche Herstellung von Rotigotinpflastern, die o.g. Ausführungsform entsprechen, wird beispielsweise in der WO 02/089778 und der WO 04/012730 beschrieben. Der Wirkstoff Rotigotin ist dabei in nicht kristalliner Form in einer silikonkleberhaltigen Schicht enthalten. Vor dem Einbringen von Rotigotin in die Kleberschicht wird Rotigotin in einem Lösungsmittel gelöst, die lösungsmittelhaltige, mit Wirkstoff beladene Klebermasse wird in einem nachfolgenden kontinuierlichen Beschichtungsprozess auf eine Release-Liner-Folie, eine Polyesterfolie, die auch als "Release-liner" oder "Schutzfolie" bezeichnet wird, aufgebracht und das Lösungsmittel durch Erwärmen in einem Trockenkanal entfernt. Nach dem Trocknungsprozess, wird auf die verbleibende offene Grenzfläche der Rotigotin-haltigen Kleberschicht eine für den Wirkstoff undurchlässige Trägerschicht aufkaschiert. Das so hergestellte Laminat wird anschließend durch mechanische Vereinzelung in einzelne Pflaster aufgeteilt.

Die Trennkanten zwischen den einzelnen Pflastern werden herkömmlich durch mechanische Vereinzelung, zum Beispiel durch Schneiden oder Ausstanzen erzeugt. Die Release-Liner-Folie kann darüber hinaus häufig mit einem S-förmigen Schnitt, dem so genannten "S-Cut", versehen werden. Dieser Schnitt erleichtert ein Ablösen der Release-Liner-Folie von dem Pflaster, um das Pflaster mit seiner Rotigotin-haltigen Kleberschicht auf die Haut des Patienten aufzukleben. Der S-Cut kann dabei einerseits als entlang des gesamten Pflasters durchgehender Schnitt oder auch als "Sollbruchstelle" in Form einer gezielten Schwächung der Release-Liner-Folie entlang einer S-Cut-Linie verwirklicht werden. Dieser "Cut" oder "Sollbruchstelle" kann neben der oben beschriebenen Form andere Formen aufweisen, beispielsweise in Form einer Geraden oder als Zick-Zack-Linie. Ein mögliches Herstellungsverfahren für ein transdermales Pflaster wird beispielsweise in der WO 04/012730, als Beispiel 1, beginnend auf Seite 14 detailliert beschrieben.

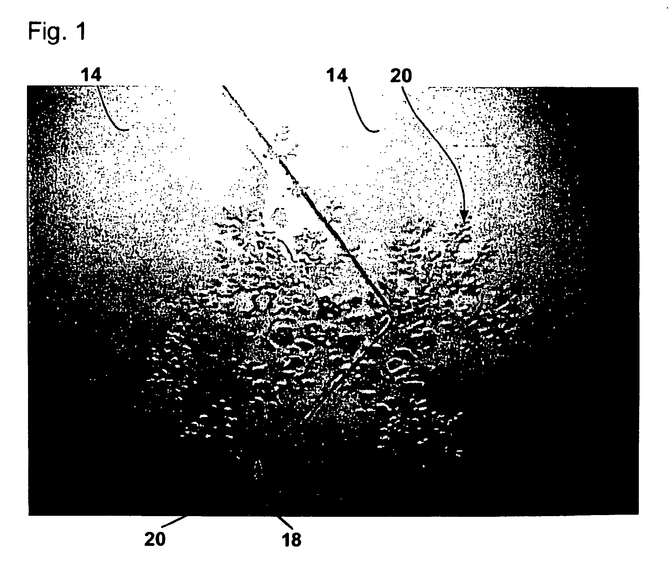

Bei einer Lagerung der so erzeugten Pflaster bei Raumtemperatur besteht jedoch die Gefahr, dass sich Rotigotin-Kristalle in der Wirkstoffschicht im Bereich der Trennkanten ausbilden, und ausgehend von den von den Kanten des Pflasters bzw. dem S-Cut der Release-Liner-Folie in Richtung des Inneren der Wirkstoffschicht, also von der entsprechenden Kante weg, aber auch entlang der Kante ausbreiten. Fig. 1 zeigt eine Trennkante 18 eines Rotigotin-haltigen Pflasters, von der sich Kristalle 20 in die Wirkstoffschicht 14 ausbreiten. Rotigotinekristalle können unterschiedliche Polymorphe bilden, die sowohl allein als auch im Gemisch auftreten können (z. B. Form I und Form II). Üblicherweise bilden die entstehenden Kristalle ein thermodynamisch stabiles Polymorph II ("Form II") von Rotigotin. Die Bildung eines solchen kristallinen Polymorphs ist in einer Pflasterformulierung unerwünscht; daher wurden bereits Anstrengungen unternommen, eine solche Kristallisation zu vermeiden. Hierzu wird bisher in einigen Fällen eine kontinuierliche Kühlung der Pflaster angewendet, um das Wachstum der Kristalle zu hemmen. Dies erfordert jedoch hohen logistischen und finanziellen Aufwand zur Aufrechterhaltung der Kühlkette von der Herstellung der Pflaster bis zu ihrer Anwendung beim Patienten, und ist in einigen Ländern nur schwer umsetzbar. Zudem wird auch die Mitarbeit jedes Patienten benötigt und damit die Handhabung für die Patienten erschwert.

Auch bei Pflastern mit anderen Wirkstoffen stellt eine solche Kristallisation des Wirkstoffs in der Nähe der gegenüber der Umgebung offenen Matrixrändern ein Problem dar. Dies gilt insbesondere für silkonbasierte Pflaster, deren Matrix eine hohe Durchlässigkeit für Wasserdampf und Sauerstoff besitzt, und für lipophile, insbesondere schlecht wasserlösliche Wirkstoffe.

### Darstellung der Erfindung

Die Aufgabe der vorliegenden Erfindung liegt daher darin, ein transdermales Pflaster zu entwickeln, bei dem die Entstehung von Kristallen in der Wirkstoffschicht erschwert und möglichst verhindert wird.

Die Lösung der Aufgabe wird durch ein transdermales Pflaster gegeben, umfassend eine Release-Liner-Folie,
eine wirkstoffhaltige Polymerschicht mit einem amorphen Wirkstoff, und
eine Trägerschicht,
wobei die.Wirkstoffschicht zwischen der Release-Liner-Folie und der Trägerschicht eingebracht ist, wobei
die Release-Liner-Folie und die Trägerschicht über eine Länge von mindestens 70% der umfangseitigen Trennkanten, thermisch und voneinander lösbar derart aneinander gebunden sind, dass die Wirkstoffschicht dadurch versiegelt ist, und wobei der Wirkstoff Rotigotin oder eines seiner pharmakologisch akzeptablen Salze ist.

Die Release-Liner-Folie wird bei der Applikation des Pflasters vor dem Aufbringen auf die Haut des Patienten abgezogen und ist daher leicht von der üblicherweise selbst klebenden Wirkstoffschicht ablösbar. Im auf die Wirkstoffschicht aufgebrachten Zustand stellt die Release-Liner-Folie eine Schutzschicht für den Wirkstoff und die diesen enthaltene Wirkstoffschicht dar. Während der Herstellung des Pflasters dient die Release-Liner-Folie aufgrund ihrer physikalischen Eigenschaften (wie z.B. Formstabilität und Reißfestigkeit unter Zugbeanspruchung) bevorzugt als Träger für die Wirkstoffschicht. Die Release-Liner-Folie stellt auch sicher, dass der Wirkstoff während der Lagerung in einer gewünschten hohen Konzentration in der Wirkstoffschicht verbleibt und sich nicht vor der Anwendung des Pflasters verflüchtigt. Die Release-Liner-Folie nimmt damit sowohl eine Schutzfunktion als auch eine Versiegelungsfunktion für die Wirkstoffschicht wahr und kann vor der Anwendung des Pflasters abgezogen werden.

Die Wirkstoffschicht enthält einen Wirkstoff, der aufgrund seiner physikochemischen Eigenschaften zur transdermalen Verabreichung geeignet ist. Im allgemeinen sind geeignete Wirkstoffkandiaten lipophil und nur begrenzt in Wasser löslich. Nimmt die Wirkstoffschicht bei Lagerung Feuchtigkeit auf, was z.B. in Gegenwart hygroskopischer Hilfstoffe möglich ist, kann die Löslichkeitsgrenze des lipophilen Wirkstoffes im Pflaster überschritten werden, mit der Folge von Kristallkeimbildung und Kristallwachstum. Dieses Risiko ist insbesondere bei lipophilen Wirkstoffen mit schlechter Wasserlöslichkeit ausgeprägt. Unter "schlecht wasserlöslichen Wirkstoffen" werden in dieser Anmeldung Wirkstoffe verstanden, die bei einem pH von 7 bei 15°C eine Wasserlöslichkeit von höchstens 1 mg/mL haben. Beispiele für solche Wirkstoffe sind Estradiol, Buprenorphin, Fentanyl, Norethindroneacetate, und insbesondere Rotigotin und seine Salze. Vorteilhafter Weise liegt der Wirkstoff, z.B. Rotigotin in der Wirkstoffschicht im Wesentlichen in Form der Wirkstoffbase und in 1-20 Gew% vor.

Dabei ist es denkbar, dass die Wirkstoffschicht gleichsam als Klebeschicht für das Pflaster dient. Daneben ist es jedoch auch möglich, dass eine separate Wirkstoffschicht, die keine Klebefunktion wahrnimmt, und eine zusätzliche Klebeschicht vorhanden sind.

Die Trägerschicht kann auch als Rückschicht bezeichnet werden und ist wirkstoffundurchlässig. Die Rückschicht dient in aufgeklebtem Zustand des transdermalen Pflasters dem Schutz des Wirkstoffs vor einer Verflüchtigung und dient zugleich als Trägerschicht für den Wirkstoff, nachdem die Release-Liner-Folie von dem Pflaster abgezogen wurde. Sowohl die Release-Liner-Folie, als auch die Trägerschicht liegen bevorzugt in Folienform vor.

Die Release-Liner-Folie und die Trägerschicht weisen zumindest eine Trennkante auf, die einen umfangseitigen Rand des Pflasters definiert (nachfolgend "umfangseitige Trennkante"), und die thermisch bewirkt ist.

In einer bevorzugten Ausführungsform werden mindestens etwa 60%, 70%, 75%, 80%, 90%, 95% oder 99% der Gesamttrennkantenlänge des umfangseitigen Rands des Pflasters thermisch bewirkt. Besonders bevorzugt werden alle umfangsseitigen Trennkanten des Pflasters vollständig thermisch bewirkt.

Üblicherweise bedeutet thermisch bewirkt in dieser Anmeldung, daß ein bestimmter in dieser Anmeldung jeweils näher beschriebener Prozess im Wesentlichen durch Einsatz von Wärme oder Hitze durchgeführt wird. Beispielsweise kann eine Trennkante im Wesentlichen durch Einwirkung von Wärme oder Hitze, beispielsweise durch einen erhitzten Draht, eine heiße Stanze etc. erzeugt werden. In einem anderen Beispiel kann das voneinander lösbare Verbinden der umfangseitigen Trennkanten im Wesentlichen durch Einwirkung von Wärme oder Hitze erzeugt werden, beispielsweise durch Schweißen, thermisches Verstemmen und/oder thermisches (Ver)Fügen. In einer Ausführungsform erfolgt das voneinander lösbare Verbinden durch Schweißen. In einem Beispiel wird das voneinander lösbare Verbinden der umfangseitigen Trennkanten der Release-Liner-Folie und der Trägerschicht im Wesentlichen durch Einwirkung von Wärme oder Hitze erzeugt, beispielsweise durch Schweißen.

Das thermische Bewirken und voneinanderlösbare Verbinden der umfangseitigen Trennkanten erfolgt üblicherweise ohne Einsatz von Klebern, die zwischen die Release-Liner-Folie und die Trägerschicht eingebracht werden. Das thermische Bewirken und voneinanderlösbare Verbinden der umfangseitigen Trennkanten erfolgt in einem anderen Beispiel üblicherweise ohne Einsatz von nichtmetallischen Stoffen, die Release-Liner-Folie und die Trägerschicht durch Flächenhaftung (Adhäsion) und innere Festigkeit (Kohäsion) verbinden und die zwischen die Release-Liner-Folie und die Trägerschicht eingebracht werden.

Das thermische Bewirken der kann beispielsweise durch Schweißen, thermisches Verstemmen und/oder thermisches (Ver)Fügen erfolgen. In einer Ausführungsform erfolgt das thermische Bewirken durch Schweißen.

Eine Trennkante im Sinne der Erfindung kann dabei einerseits zur Vereinzelung des Pflasters dienen und somit den umfangseitigen Rand des Pflasters oder Teile davon bilden. Daneben kann als Trennkante auch ein innerhalb des Randes des Pflasters, also auf einer Schicht eines vereinzelten Pflasters vorgenommener teilweiser Schnitt oder eine gezielte Schwächung einer der Schichten verstanden werden (nachfolgend "Sollbruch-Trennkante" oder "Sollbruchkante"). Die Trennkante kann also einerseits eine umfangseitige Kante sein, an der ein Pflaster von einem anderen Pflaster oder einem Rohling getrennt ist, andererseits kann die Trennkante auch eine Sollbruch-Trennkante sein, die zum Auftrennen einer Schicht, z.B. der Release-Liner-Folie, zur Benutzung des Pflasters dient. "Trennen" wird somit als quantitatives Teilen und somit Gegenstück zu "Fügen" verstanden.

Bevorzugt werden die Release-Liner-Folie und die Trägerschicht zumindest entlang einer der umfangseitigen Trennkanten des Pflasters thermisch und voneinander lösbar aneinander gebunden. Bevorzugt wird insbesondere, dass sie derart aneinander gebunden sind, dass die Wirkstoffschicht dadurch versiegelt ist. In einer bevorzugten Ausführungsform sind die Release-Liner-Folie und die Trägerschicht mindestens entlang 60%, 70%, 75%, 80%, 90%, 95% oder 99% der Gesamtlänge der umfangseitigen Trennkanten thermisch und voneinander lösbar aneinander gebunden.

Eine durchgehende "umfangsseitige Trennkante" entlang des Umfangs des Pflasters, beispielsweise bei einem kreis- oder ellipsenförmigen Pflaster kann dabei als mehrere Trennkanten angesehen werden, bei denen die Übergänge zwischen den einzelnen Trennkanten kontinuierlich sind.

Besonders bevorzugt sind Release-Liner-Folie und die Trägerschicht über die gesamte Länge der umfangseitigen Trennkanten thermisch und voneinander lösbar aneinander gebunden, so dass die thermische Verbindung der Release-Liner-Folie und der Trägerschicht entlang der umfangseitigen Trennkante die Wirkstoffschicht entlang dieser Trennkante einschließt. In einem solchen Fall kann die Wirkstoffschicht im Wesentlichen gegenüber der Umwelt abgeschlossen und dadurch versiegelt sein. Der Begriff "im Wesentlichen gegenüber der Umwelt abgeschlossen" bedeutet, dass der Gas- und Wirkstoffaustausch zwischen der Wirkstoffschicht und der Pflasterumgebung im Gegensatz zu den aus dem Stand der Technik bekannten Verfahren auch im Kantenbereich in dem Maß unterbunden ist, wie es sich aus den Materialeigenschaften der Träger - und Release Liner Folien ergibt. Üblicherweise werden Träger - und Release Liner Folien verwendet, die keinen Wirkstoffdurchtritt und nur einen geringen Gasaustausch erlauben.

Dass die Release-Liner-Folie und die Trägerschicht voneinander lösbar aneinander gebunden sind, bedeutet, dass die Bindung zwischen den beiden Schichten so stark ist, dass die Release-Liner-Folie an der Trägerschicht haftet und an dieser nach der Produktion und während Transport und Lagerung entlang der umfangseitigen Trennkante über einen Siegelrand gebunden ist, dass die beiden Schichten jedoch bei einer herkömmlichen Verwendung des Pflasters durch Abziehen der Release-Liner-Folie voneinander gelöst werden können, d.h., dass weder die Release-Liner-Folie noch die Trägerschicht beim Abziehen der Release-Liner-Folie von der Trägerschicht zerstört werden. Zu diesem Zweck werden der von den zur thermischen Bewirkung verwendeten Werkzeugen ausgeübte Druck sowie die Breite des durch die thermische Bewirkung entstehenden Siegelrandes in Abhängigkeit von den jeweiligen Verhältnissen im Pflaster, wie Dicke und Material der Trägerschicht, Matrix und Release-Linerschicht eingestellt. Beispielsweise kann der die Release-Liner-Folie und die Trägerschicht verbindende Siegelrand eine Breite von 50-1000 µm, insbesondere von 100-300 µm haben.

Um bei der thermischen Bewirkung der umfangseitigen Schnittkante eine Verdrängung der Wirkstoffmatrix aus dem Bereich des Siegelrands zu ermöglichen, umfasst die Wirkstoffmatrix vorteilhafterweise ein Polymer, das günstige Fließeigenschaften und eine niedrige Oberflächenspannung hat. Silikonbasierte Polymere, insbesondere drucksensitive Polyorganosiloxankleber, wie z.B. Dimethylsiloxane, erfüllen diese Bedingungen in besonderem Maße, da sie sich auf Grund ihrer viskoelastischen Eigenschaften leicht aus dem Bereich des Siegelrands verdrängen lassen. Dies ist insbesondere in einer bevorzugten Ausführungsform der Fall, wenn die Materialien der Release-Liner-Folie und Trägerschicht silikonisiert sind. Die Wirkstoffschicht hat dabei bevorzugt eine Dicke von 35-120 µm, besonders bevorzugt von 40-80 µm und ganz besonders bevorzugt von 45-60µm. In einer bevorzugten Ausführungsform der Erfindung erfolgt die Verdrängung der Wirkstoffmatrix aus der vorgesehenen umfangseitigen Trennkante während des thermischen Vereinzelungsschrittes. Denkbar ist allerdings auch, dass in einem ersten Schritt eine Trennkantenkontur durch Druckeinwirkung vorgegeben wird, und in einem zweiten Schritt die thermisch bewirkte Vereinzelung und Versiegelung an der vorbewirkten Trennkantenkontur erfolgt. Dieses zweistufige Verfahren kann insbesondere von Vorteil sein, wenn der Wirkstoff im Pflaster thermisch labil ist und vorab aus dem thermisch zu bewirkenden Schnittkantenbereich verdrängt werden soll und/oder wenn die Wirkstoffmatrix sich in einem einzigen Verarbeitungsschritt nur schwer aus dem Bereich des vorgesehenen Siegelrandes verdrängen lässt.

In den hydrophoben, vorzugsweise silikonbasierten Klebern können neben dem Wirkstoff noch andere Hilfsstoffe, wie z.B. Antioxidanzien wie Natriumbisulfit oder alpha-Tocopherol, Penetrationsbeschleuniger oder Kristallisationsinhibitoren wie Polyvinylpyrrolidon (z. B. Kollidon®, BASF AG), Polyethylenglycol, Polypropylenglycol, Glycerol und Glycerol-Fettsäureester, sowie Copolymere aus Vinylacetat mit Ethylen oder PVP enthalten sein.

Mit Vorteil weist zumindest die Release-Liner-Folie als Sollbruchkante einen geschwächten und/oder zumindest teilweise unterbrochenen Bereich auf, der als Öffnungshilfe für das Pflaster verwendbar ist. Eine derartige Sollbruch-Trennkante dient dem Trennen der Release-Liner-Folie in zwei Teile während der Benutzung des Pflasters entlang einer definierten Kante. Hierfür kann die Release-Liner-Folie geschwächt sein, ohne dass eine Versiegelung der Wirkstoffschicht nach außen im Fall einer thermischen Bindung zwischen der Trägerschicht und der Release-Liner-Folie dadurch gestört wird. Eine teilweise Unterbrechung der Release-Liner-Folie entlang der Sollbruch-Trennkante der Release-Liner-Folie kann dabei derart bemessen sein, dass die Versiegelungswirkung der Wirkstoffschicht kontrolliert bleibt. Der geschwächte und/oder zumindest teilweise unterbrochene Bereich bildet dabei eine Sollbruch-Trennkante, die bevorzugt keinen umfangseitigen Rand des Pflasters definiert, sondern die im Allgemeinen entlang eines Wegs auf dem Pflaster verläuft. Die Sollbruch-Trennkante verläuft dabei typischerweise in der Ausdehnungsebene des Pflasters entlang einer Gerade.

In einer bevorzugten Ausführungsform sind die Release-Liner-Folie und die Trägeschicht aus demselben Material gefertigt. Beispiele für solche Materialien sind Polyethylen, Polypropylen sowie Polyethylenterephthalat. Ein Vorteil der Fertigung der Release-Liner-Folie und der Trägerschicht aus demselben Material besteht beispielsweise darin, dass die thermischen Eigenschaften hinsichtlich ihrer Festigkeit sowie die Dichtungseigenschaften der Release-Liner-Folie und der Trägerschicht, abgesehen von möglicherweise unterschiedlichen Stärken der einzelnen Schichten, gleich sind. Dies führt zu einer vereinfachten Verarbeitbarkeit, insbesondere kann für beide Schichten eine thermisch bewirkte umfangseitige Trennkante erzeugt werden, die für beide Schichten gleich verläuft. Vorzugsweise werden Release-Liner-Folie und Trägerschicht aus einem Material gefertigt, dessen Schmelztemperatur in einem Temperaturbereich liegt, bei dem der im Pflaster enthaltene Wirkstoffs noch keine thermisch-chemische Zersetzung oder Umwandlung eingeht.

In einer bevorzugten Ausführungsform weist die Release-Liner-Folie und die Trägerschicht eine vordefinierte Stärke oder Dicke zur Steuerung eines Gasdurchflusses durch diese Schicht auf. Je nach Material, aus dem die Release-Liner-Folie und die Trägerschicht hergestellt sind, kann jede dieser Schichten einen gewissen Gasaustausch zwischen der Wirkstoffschicht und der Umgebung ermöglichen. Typische Stärken der Release-Liner-Folie liegen bei etwa 50-150 µm und der Trägerschicht zwischen etwa 15 µm und etwa 30 µm. Eine größere Stärke der Schichten führt dabei zu einem verringerten Gasaustausch. Bei der Wahl der Stärke der Schicht ist zur Regelung des Gasdurchflusses auch eine Porosität des Materials der jeweiligen Schicht zu beachten, die auch bevorzugt vordefiniert und besonders bevorzugt für die Release-Liner-Folie und Trägerschicht gleich ist. Daneben ist es auch denkbar, dass das Material der Trägerschicht und der Release-Liner-Folie auf ein gewünschtes Maß eines Gasdurchflusses angepasst wird.

Bei einem erfindungsgemäßen Verfahren zur Herstellung eines transdermalen Pflasters ist zwischen einer Release-Liner-Folie und einer Trägerschicht eine Wirkstoffschicht z.B. mit Rotigotin und einem seiner pharmakologisch akzeptablen Salze als Wirkstoff angeordnet, wobei die Release-Liner-Folie und die Trägerschicht des Pflasters mit zumindest einer thermisch bewirkten umfangseitigen Trennkante versehen wird, um Kristallisation im Bereich dieser Trennkante zu verhindern. Bevorzugt wird die Release-Liner-Folie und die Trägerschicht des Pflasters mit mehreren umfangseitigen und über ihre gesamte Länge thermisch bewirkten Trennkanten versehen.

Dadurch, dass die Trennkante thermisch bewirkt wird, können sowohl Kristallisationskeime als auch andersartige Keime unterbunden bzw. abgetötet werden. Somit kann insbesondere die Entstehung von Kristallen bereits bei der Herstellung des Pflasters verhindert werden.

Bei einem bevorzugten Verfahren zur Herstellung eines transdermalen Pflasters werden zwischen einer Release-Liner-Folie und einer Trägerschicht eine Wirkstoffschicht mit Rotigotin und einem seiner pharmakologisch akzeptablen Salze als Wirkstoff angeordnet, wobei die Release-Liner-Folie und die Trägerschicht des Pflasters mit zumindest einer thermisch bewirkten umfangseitigen Trennkante, bevorzugt mit über ihre gesamte Länge thermisch bewirkten umfangseitigen Trennkanten versehen wird, und wobei die Release-Liner-Folie und die Trägerschicht entlang der umfangseitigen Trennkanten über eine Länge von mindestens 70% der umfangseitigen Trennkanten, thermisch miteinander verbunden werden, so dass die Wirkstoffschicht durch die thermisch miteinander verbundenen Schichten versiegelt ist

Bevorzugt wird die Release-Liner-Folie und die Trägerschicht über die gesamte Länge der umfangseitigen Trennkanten thermisch miteinander verbunden.

Bevorzugt ist es also möglich, dass die umfangseitige Trennkante gleichzeitig einen Siegelrand zwischen der Trägerschicht und der Release-Liner-Folie bildet. Das Verbinden der Release-Liner-Folie mit der Trägerschicht entlang der umfangseitige Trennkante erfolgt dabei im Wesentlichen während desselben Vorgangs, der auch die umfangseitige Trennkante selbst realisiert. Beispielsweise ist es möglich, dass durch Verwendung eines Paars gegenläufiger, beheizbarer Walzen oder ähnlicher Werkzeuge einerseits eine umfangseitige Trennkante thermisch bewirkt und andererseits die Release-Liner-Folie mit der Trägerschicht thermisch miteinander verbunden wird. Die Release-Liner-Folie und die Trägerschicht werden derart miteinander verbunden, dass sie danach voneinander lösbar sind, d.h., dass die Release-Liner-Folie von der Trägerschicht ablösbar ist, ohne dass eine der beiden Schichten dabei zerstört werden muss.

Bevorzugt wird die Release-Liner-Folie in einem Bereich einer Sollbruchkante, die als Öffnungshilfe für das Pflaster verwendbar ist, thermisch geschwächt und/oder zumindest teilweise unterbrochen. Die Sollbruch-Trennkante, die als Öffnungshilfe für das Pflaster verwendbar ist, liegt im Inneren des Pflasters, d.h., innerhalb des umlaufenden Randes, der durch eine thermisch bewirkte umfangseitige Trennkante gebildet werden kann. Bei der thermisch geschwächten und/oder zumindest teilweise unterbrochenen Sollbruch-Trennkante kann es sich beispielsweise um eine Öffnungshilfe entsprechend dem so genannten S-Cut handeln.

Dabei wird zumindest eine der thermischen Behandlungen oberhalb einer Schmelztemperatur der jeweiligen Wirkstoffkristalle durchgeführt. Die thermischen Behandlungen bezeichnen dabei einerseits das Versehen des Pflasters mit zumindest einer thermisch bewirkten Trennkante und andererseits das Verbinden der Release-Liner-Folie mit der Trägerschicht entlang der umfangseitigen Trennkante. Im Beispiel von Rotigotin als Wirkstoff in der Wirkstoffschicht liegt der Schmelzpunkt des entsprechenden höher schmelzenden Rotigotinkristalls ("Form II") bei 97°C ± 2°C. Um die Schneidwerkzeuge kristallkeimfrei zu halten wird daher bevorzugt, diese auf eine höhere Temperatur als den Schmelzpunkt des jeweiligen Wirkstoffkristalls, beispielsweise also auf mindestens 100°C, vorzugsweise auf über 110 °C oder über 120°C im Fall von Rotigotin zu bringen.

Bevorzugt wird ein Verfahren, bei dem zumindest eine der thermischen Behandlungen oberhalb einer Schmelztemperatur der Wirkstoffkristalle von Rotigotin Form II durchgeführt wird.

Besonders bevorzugt wird ein Verfahren, bei dem die thermische Behandlung oberhalb einer Temperatur von 120°C durchgeführt wird.

Zudem muss die Temperatur so hoch gewählt werden, dass Sie eine thermische Bewirkung der Schnittkanten und bevorzugt ein Verschmelzen der Release-Liner-Folie mit der Trägerschicht gewährleistet. Die hierfür erforderlichen Temperaturen hängen von der Art der für die Release-Liner-Folie und die Trägerschicht verwendeten Materialien ab und betragen z.B. für Polyethylenfolien ca 100-120°C und mehr, für Polypropylenfolien etwa 165°C und mehr sowie für Polyethylenterephtalat ca 260°C und mehr. Dabei werden die Materialien der Release-Liner-Folie und der Trägerschicht bevorzugt so gewählt, dass die Schmelztemperaturen besagter Folien unterhalb der Zersetzungstemperatur des jeweils im Pflaster vorliegenden Wirkstoffes liegt.

Mit Vorteil sind die Release-Liner-Folie und die Trägerschicht mit der Wirkstoffschicht versehen und die Schichten werden mit der Wirkstoffschicht durch ein Paar beheizbare gegenläufige Walzen, die als Walzenschleuse fungieren, durchgeführt. Bei diesem Verfahren könnte die Pflasteroberfläche auf der Oberfläche einer der gegenläufigen Walzen als beheizbare Struktur ausgeprägt sein. Eine der beiden Walzen kann dazu dienen, umfangseitige Trennkanten zu erzeugen, insbesondere einzelne Pflaster aus den Schichten zu vereinzeln. Dagegen kann die andere der beiden Walzen die Struktur der Sollbruch-Trennkante, z.B. in Form eines "S-Cuts" oder einer entsprechenden, ähnlichen Öffnungshilfe tragen. Hierbei ist es nicht unbedingt notwendig, dass beide Walzen beheizbar sind. Es kann auch die Walze zur Erzeugung einer thermisch bewirkten umfangseitigen Trennkante beheizt sein, während die andere Walze zur Erzeugung der Struktur der Sollbruch-Trennkante (z.B. des S-Cuts) nur eine gezielte Schwächung der Release-Liner-Folie bewirken soll und nicht beheizbar sein muss, wenn sie nicht mit der Wirkstoffschicht in Kontakt kommt. Bevorzugt ist jedoch, dass sowohl eine Walze zur Erzeugung einer umfangseitigen Trennkante als Rand des Pflasters als auch eine Walze zur Erzeugung einer Sollbruch-Trennkante mit einem geschwächten und/oder zumindest teilweise unterbrochenen Bereich, insbesondere als Öffnungshilfe für das Pflaster, beheizbar sind. Es ist schließlich auch möglich, dass sowohl eine umfangseitige Trennkante als Rand des Pflasters als auch eine Sollbruch-Trennkante mit geschwächtem und/oder zumindest teilweise unterbrochenem Bereich durch eine einzige Walze, die beheizbar ist, bewirkt werden, während eine gegenläufige Gegenwalze unbeheizt sein kann.

Ein weiterer Gegenstand der Erfindung ist ein Verfahren zur Herstellung eines transdermalen Pflasters, wobei zwischen einer Release-Liner-Folie und einer Trägerschicht eine Wirkstoffschicht mit einem schlecht wasserlöslichen Wirkstoff, insbesondere mit Rotigotin und einem seiner pharmakologisch akzeptablen Salze als Wirkstoff, ganz besonders bevorzugt mit der freien Base von Rotigotin angeordnet ist, wobei das Verfahren dadurch charakterisiert ist, dass unmittelbar bevor die Release-Liner-Folie und die Trägerschicht mit einer umfangseitigen Trennkante versehen wird, die Release-Liner-Folie und die Trägerschicht elektrisch entladen werden. Durch das Versehen der Release-Liner-Folie und der Trägerschicht mit der thermisch bewirkten umfangseitigen Trennkante kann dabei insbesondere eine Vereinzelung der Pflaster durchgeführt werden. Durch das elektrische Entladen der Release-Liner-Folie und der Trägerschicht ist eine weitere Reduktion des Risikos der Wirkstoffkristallisation in der Wirkstoffschicht möglich. Der Ausdruck "unmittelbar bevor die Release-Liner-Folie und die Trägerschicht mit einer umfangseitigen Trennkante versehen wird" bedeutet dabei, dass auf die entsprechende Schicht zwischen dem elektrischen Entladen und dem Versehen mit einer umfangseitigen Trennkante kein weiterer Verfahrensschritt angewendet wird und dass diese Schicht zwischen dem Entladen und dem Versehen mit einer umfangseitigen Trennkante keine unnötige Wegstrecke zurücklegt. Dies hat den Vorteil, dass zwischen dem Entladen und dem Versehen der Schicht mit einer umfangseitigen Trennkante kein erneutes Aufladen einer der Schichten erfolgen kann. Für die Entladung der Release-Liner-Folie und der Trägerschicht unmittelbar vor der Vereinzelung bzw. dem Versehen mit einer umfangseitigen Trennkante kann das Abführen der Spannung beispielsweise über eine großflächige elektrische Seite erfolgen. Die Spannung kann zum Beispiel über Carbonfäden oder Edelstahldrähte abgeführt werden. Die Spannungsableitung ist dabei bevorzugt mit dem thermischen Vereinzelungsverfahren kombiniert. In einer bevorzugten Ausführungsform werden daher Release-Liner-Folie und Trägerschicht beide jeweils elektrisch entladen und sodann jeweils mit einer thermisch bewirkten umfangseitigen Trennkante versehen und voneinander lösbar aneinander gebunden, so dass die zwischenliegende wirkstoffhaltige Schicht versiegelt ist.

Bei einem ferner bevorzugten Verfahren zur Herstellung eines transdermalen Rotigotinpflasters liegt der Wirkstoff in nicht kristalliner Form vor. Bei einem anderen bevorzugten Verfahren zur Herstellung eines transdermalen Rotigotinpflasters liegt der Wirkstoff in amorpher Form vor Mit Vorteil liegt Rotigotin in der Wirkstoffschicht im Wesentlichen, d.h zu über 90 Mol% in Form der Wirkstoffbase und in 5-20 Gew% bezogen auf das Gewicht der Wirkstoffschicht vor.

Ein weiterer Gegenstand der vorliegenden Erfindung ist somit auch ein transdermales Pflaster, bei dem Rotigotin in der Wirkstoffschicht zu über 90 Mol% in Form der.Wirkstoffbase und in 5-20 Gew% bezogen auf das Gewicht der Wirkstoffschicht vorliegt. -In dem somit hergestellten Rotigotinflaster sind auch nach einer Lagerung von 12 Monaten bei 25°C, bevorzugt bei 24 Monaten bei 25°C keine Kristalle von Rotigotin (z.B. Form II) nachweisbar. Dass der Wirkstoff in amorpher Form vorliegt bedeutet, dass er im Wesentlichen keine Kristallstrukturen aufweist.

Das erfindungsgemäße Verfahren dient zur Herstellung eines transdermalen Pflasters, bei dem zwischen einer Release-Liner-Folie und einer Trägerschicht eine Wirkstoffschicht angeordnet ist, die einen nicht-kristallinen, schwer wasserlöslichen Wirkstoff, insbesondere Rotigotin oder eines seiner pharmakologisch akzeptablen Salze enthält und im Wesentlichen frei ist von Kristallisationskeimen des Wirkstoffs. In einer Ausführungsform enthält die Wirkstoffschicht einen amorphen, schwer wasserlöslichen Wirkstoff, insbesondere Rotigotin oder eines seiner pharmakologisch akzeptablen Salze. Die Release-Liner-Folie und die Trägerschicht des Pflasters werden dabei mit zumindest einer thermisch bewirkten Trennkante versehen. Der Ausdruck "im Wesentlichen" bringt dabei zum Ausdruck, dass Kristallstrukturen bis zu einem Anteil von weniger als 1 Gew% bezogen auf den jeweils eingesetzten Wirkstoffgehalt vorhanden sein dürfen, ohne, dass der Wirkstoff als in zumindest teilweise kristalliner Form vorliegend bezeichnet wird.

Gegenstand der Erfindung ist auch die Verwendung eines oben beschriebenen Verfahrens zur Unterdrückung des Auftretens von Rotigotinkristallen im Pflaster.

Gegenstand der Erfindung ist ferner ein Verfahren umfassend die Verwendung eines Werkzeug, welches zwei gegenläufige, zumindest teilweise beheizbare Walzen umfasst, durch welche die Release-Liner-Folie und die Trägerschicht sowie die Wirkstoffschicht durchführbar sind.

In einem bevorzugten Verfahren ist dabei eine erste Walze zur Vereinzelung des Pflasters eingerichtet und eine zweite, zur ersten gegenläufige Walze zur Ausbildung eines geschwächten und/oder zumindest teilweise unterbrochenen Bereichs eingerichtet.

Erfindungsgemäß umfasst das Werkzeug zwei gegenläufige, zumindest teilweise beheizbare Walzen, durch welche die Release-Liner-Folie und die Trägerschicht sowie die Wirkstoffschicht durchführbar sind. Die Wirkstoffschicht kann dabei auf der Release-Liner-Folie und der Trägerschicht aufgebracht sein. Der Ausdruck "durchführbar" bedeutet hierbei, dass die entsprechende Schicht zwischen den zwei gegenläufigen Walzen, die eine Walzenschleuse bilden, hindurchgeführt werden kann. Zumindest eine der beiden Walzen ist dabei zumindest teilweise beheizbar, um zumindest eine Trennkante des Pflasters thermisch zu bewirken. Dabei ist es denkbar, dass die Walze nur im Bereich eines Werkzeugelements beheizbar ist, welches zur Ausprägung einer umfangseitigen Trennkante geeignet ist. Die Geometrie des zur Ausprägung der umfangseitigen Trennkante geeigneten Werkzeugs, das als Siegelelement zur Versiegelung der Wirkstoffschicht wirkt ist bevorzugt derart gewählt, dass die Flussrichtung der Wirkstoffhaltigen Matrix ins Pflasterinnere gerichtet ist. Diese bevorzugte Ausführung des Siegelelements erlaubt längere Reinigungsintervalle, eine gegebenenfalls längere Wartungsfreiheit und/oder Lebensdauer des Werkzeugs. Neben der Ausführung des Werkzeugs mit nur einem beheizbaren Werkzeugelement ist es jedoch auch möglich, dass eine Walze vollständig beheizbar ist oder dass beide Walzen teilweise bzw. vollständig beheizbar sind. Die entsprechenden Teile der Walze oder Walzen sind dabei so konstruiert, dass sie, beispielsweise durch einen elektrischen Widerstand, auf eine gewünschte Temperatur gebracht werden können, die bevorzugt oberhalb einer Schmelztemperatur eines Wirkstoffkristalls und/oder oberhalb der Sterilisationstemperatur eines bestimmten Keims liegen kann. Im Fall von Rotigotin ist es von Vorteil, wenn wenigstens die Walze, die zur Vereinzelung des Pflasters ausgebildet ist, auf mindestens 100°C, oder auf über 110°C oder über 120°C erhitzbar ist. Wenn Polypropylen bzw. Polyethylenterephtalat-Folien in der Release-Liner- und Trägerschicht zur Anwendung kommen, ist eine Erhitzung der zur Einführung der Trennkanten verwendeten Werkzeugelemente auf ca. 170°C bzw. ca. 260°C und darüber vorteilhaft.

Darüber hinaus ist es möglich, dass mehr als zwei Walzen zur Durchführung des oben beschriebenen Verfahrens zur Herstellung eines oben beschriebenen Pflasters eingesetzt werden, zum Beispiel in Form einer sequentiellen Anordnung mehrerer zueinander gegenläufiger Walzenpaare.

In einer bevorzugten Ausführungsform ist eine erste Walze zur Vereinzelung des Pflasters durch zumindest eine umfangseitige Trennkante eingerichtet, und eine zweite, zur ersten gegenläufige Walze zur Ausbildung eines geschwächten und/oder zumindest teilweise unterbrochenen Bereichs durch eine Sollbruchkante eingerichtet. Wie oben beschrieben, wird die Vereinzelung des Pflasters durch eine thermisch bewirkte umfangseitige Trennkante vollzogen, welche die Schichten durchtrennt. Die Ausbildung des geschwächten und/oder zumindest teilweise unterbrochenen Bereichs als Sollbruch-Trennkante erfolgt dagegen bevorzugt derart, dass lediglich die Release-Liner-Folie geschwächt und/oder zumindest teilweise unterbrochen wird, also nicht alle Schichten durchtrennt werden. Aus diesem Grund kann die zweite, zur Herstellung der Sollbruchkante ausgebildete Walze auch derart ausgestaltet sein, dass sie die Sollbruchkante nicht thermisch sondern rein mechanisch beispielsweise durch Schnitte bewirkt. In einer bevorzugten Ausführungsform der Erfindung ist allerdings auch die zweite Walze beheizbar.

Neben derartigen, bevorzugten Walzen kann das Werkzeug auch durch eine Stanzvorrichtung mit einem erhitzten Stanzwerkzeug gegeben sein.

In einer weiteren bevorzugten Ausführungsform des erfindungsgemäßen Verfahrens ist das Werkzeug dazu geeignet, eine Vielzahl gleichartiger transdermaler Pflaster ausgehend von jeweils einer Bahn einer Release-Liner-Folie, einer Trägerschicht und einer Wirkstoffschicht zu vereinzeln. Dieses Werkzeug dient also einer Herstellung von transdermalen Pflastern im industriellen Maßstab, bei der die Trennkanten thermisch bewirkt werden. Durch die thermische Erzeugung der Trennkanten wird die Entstehung von Keimen, insbesondere von Kristallkeimen direkt bei der Herstellung verhindert.

Bei einem oben beschriebenen transdermalen Pflaster werden ein Kristallwachstum begünstigende Einflüsse der Atmosphäre an den Pflasterkanten, beispielsweise durch Sauerstoffeintrag, Wasseraufnahme, Verdunstungsverluste oder Kontamination der Produktionsstätte durch einzelne "Impf"kristalle der "Form II" von Rotigotin bzw. anderer Wirkstoffkristalle gezielt verhindert.

Beim erfindungsgemäßen Verfahren wird eine Kontamination des Trennwerkzeugs mit einem Wirkstoffkristall, beispielsweise mit Rotigotin "Form II", und folglich eine sich ansonsten anschließende Kontamination der Trennkante, die durch das Trennwerkzeug erzeugt wird, unterbunden. Auch mechanische Scherkräfte an den mechanisch bewirkten Trennkanten und/oder elektromechanische Impulse beim Vereinzeln der Pflaster, die durch Spannungsunterschiede zwischen der Release-Liner-Folie und der Trägerschicht oder andere Effekte und anschließenden Kurzschluss beim mechanischen Vereinzeln hervorgerufen werden, werden bei einem bevorzugten Verfahren zuverlässig verhindert. Schließlich wird eine Kristallkeimbildung an Oberflächendefekten mechanisch bewirkter Trennkanten vermieden.

Es wird folglich ein transdermales Pflaster zur Verfügung gestellt, das die Entstehung von Kristallen in der Wirkstoffschicht des Pflasters bereits in seiner Herstellung verhindert bzw. deutlich reduziert.

Die erfindungsgemäßen transdermalen Pflaster sind zur Behandlung von Erkrankungen geeignet. Ist Rotigotin oder eines seiner pharmazeutisch akzeptablen Salze als Wirkstoff im Pflaster enthalten, sind die transdermalen Pflaster insbesondere zur Behandlung von Erkrankungen geeignet, die mit Störungen des Dopaminstoffwechsels und/oder Störungen der dopaminergen Signalkaskade einhergehen. Rotigotin ist daher insbesondere geeignet zur Behandlung und/oder Prävention einer Erkrankung ausgewählt aus der Gruppe
- Parkinson Plus,
- Depression,
- Fibromyalgie, sowie insbesondere
- Morbus Parkinson und
- Restless Leg Syndrom.

Weitere Vorteile und bevorzugte Ausführungsformen ergeben sich aus der Gesamtheit der Ansprüche und der nachfolgenden Beispielbeschreibung.

### Kurze Figurenbeschreibung

- Fig. 1: zeigt ein Foto eines transdermalen Pflasters aus dem Stand der Technik, bei dem entlang einer Trennkante Kristallwachstum in der Wirkstoffschicht zu beobachten ist;
- Fig. 2: zeigt ein Foto eines erfindungsgemäßen transdermalen Pflasters mit einer thermisch bewirkten Trennkante ohne Kristallwachstum in der Wirkstoffschicht; und
- Fig. 3: zeigt ein Foto eines transdermalen Pflasters, das gemäß Vergleichsbeispiel 1 mit einer Trennkante versehen wurde und Kristallwachstum aufweist.
- Fig. 4: zeigt eine Versuchsskizze zur Herstellung eines erfindungsgemäßen transdermalen Pflasters, bei der die Spannungsunterschiede ausgeglichen/abgeführt werden, d.h. die Spannungsentladung der Release-Liner-Folie und der Trägerschicht erfolgt.
- Fig. 5: zeigt ein Foto eines transdermalen Pflasters mit einer geschnittenen Trennkante, bei der während der Herstellung einer Trennkannte/Sollbruchkante die Spannungsentladung der Release-Liner-Folie und der Trägerschicht erfolgt ist, ohne Kristallwachstum in der Wirkstoffschicht.
- Fig. 6: zeigt eine Versuchsskizze gem. Vergleichsbeispiel 2 zur Herstellung eines erfindungsgemäßen transdermalen Pflasters,bei der Spannungsunterschiede angelegt worden sind und nicht ausgeglichen/abgeführt werden, d.h. die Spannungsentladung der Release-Liner-Folie und der Trägerschicht nicht erfolgt. (Das Schneidewerkzeug (Skalpell) ist nicht mit den Leitern verbunden.)
- Fig. 7: zeigt ein Foto eines transdermalen Pflasters, das gemäß Vergleichsbeispiel 2 mit einer Trennkante versehen wurde und Kristallwachstum aufweist.
- Fig. 8: zeigt ein Foto eines transdermalen Pflasters, das gemäß Vergleichsbeispiel 3 mit einer Trennkante versehen wurde und Kristallwachstum aufweist.

### Beispiele

### Beispiel 1

Aus einem Rotigotin-haltigen Pflaster, bei dem an den Schnitträndern bereits Kristallwachstum zu beobachten war, wurde aus einem kristallfreien Abschnitt mit einem Hitzedraht ein kleineres Pflaster ausgeschnitten. Der Hitzedraht war dabei Teil einer Styroporschneidemaschine. Dabei wurden die gegenüberliegenden Flächen des Pflasters, also die Release-Liner-Folie und die Trägerschicht thermisch miteinander verklebt. Das Pflaster wurde bei unterschiedlichen Temperaturen, nämlich 1 Woche bei ca. 4°C und anschließend ca. 10 Monate bei Raumtemperatur gelagert. Diese thermisch bewirkte Trennkante des Pflasters zeigte nach einer optischen Inspektion der Kante kein Kristallwachstum.

### Beispiel 2

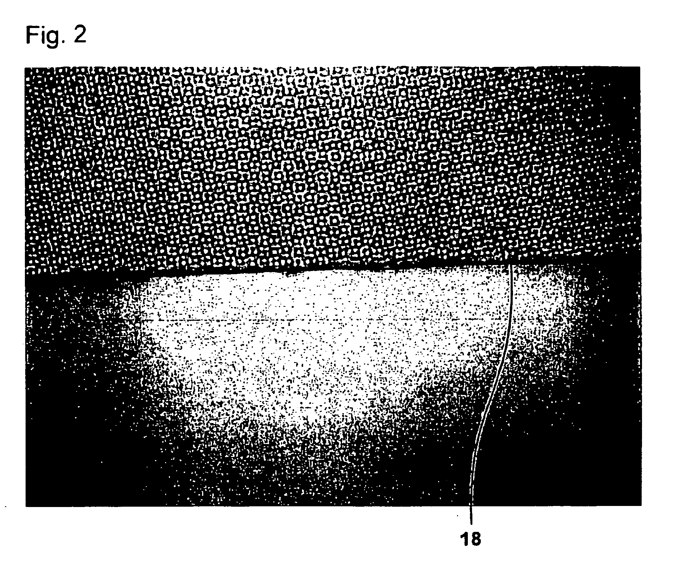

Aus einem Rotigotin-haltigen Pflaster, bei dem an den Schnitträndern bereits Kristallwachstum zu beobachten war, wurde aus einem kristallfreien Abschnitt mit einem handelsüblichen Folienschweißgerät ein kleineres Pflaster ausgelöst. Dabei wurden die gegenüberliegenden Flächen des Pflasters, also die Release-Liner-Folie und die Trägerschicht, thermisch miteinander verklebt. Das Pflaster wurde bei unterschiedlichen Temperaturen, nämlich eine Woche bei ca. 4°C und anschließend ca. 10 Monate bei Raumtemperatur gelagert. Fig. 2 zeigt ein Foto des so behandelten Pflasters in 10-facher Vergrößerung nach der oben beschriebenen Lagerung. Es ist deutlich zu erkennen, dass die optische Inspektion kein Kristallwachstum an der Trennkante 18 erkennen lässt.

### Vergleichsbeispiel 1

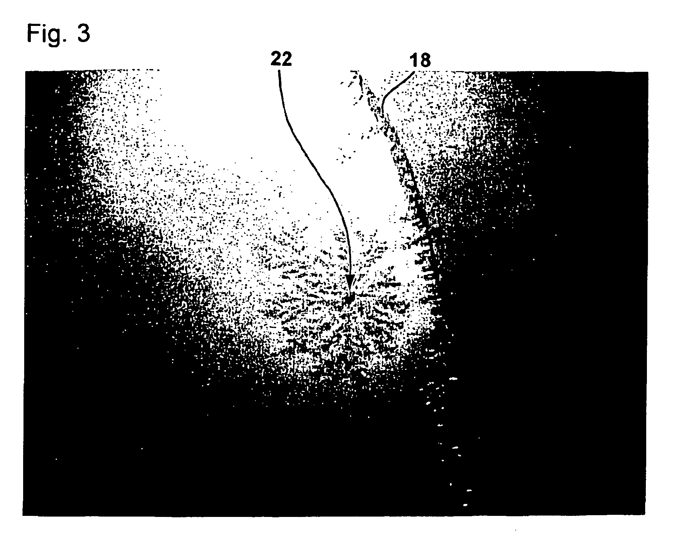

In einem kristallfreien Abschnitt eines Rotigotin-haltigen Pflasters wurde mit einer Haushaltsschere eine neue Trennkante erzeugt. Die Umgebung dieser Trennkante wurde mit einem Hochspannungsgenerator elektrischen Feldern, nämlich ca. 10.000 V ausgesetzt, um den Einfluss elektrischer Ladungen auf den Schichten auf das Kristallwachstum zu erforschen. Das Pflaster wurde bei Raumtemperatur für zwei Wochen gelagert. Wie Fig. 3 zu entnehmen ist, die ein Foto dieser Trennkante zeigt, ist ein deutlich sichtbares Kristallwachstum an der Trennkante 18 sowie an der der Funkenstrecke ausgesetzten Stelle 22 zu beobachten.

### Beispiel 3

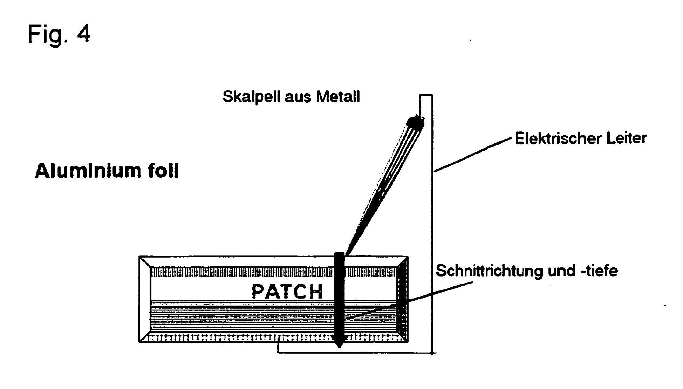

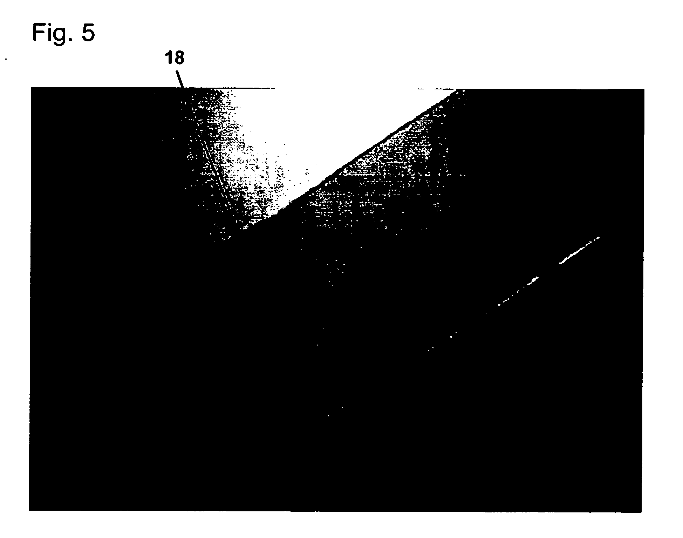

Ein Rotigotin-haltiges Pflaster wurde vollständig in eine Aluminiumfolie eingewickelt und diese anschließend über ein Potentialausgleichskabel mit einem Skalpell aus Metall elektrisch leitend verbunden(Fig. 4). Mit dem so elektrisch neutralisierten Skalpell wurde das in Aluminium eingeschlagene Pflaster an mehreren Stellen durchschnitten. Das Pflaster wurde bei Raumtemperatur 2 Monate gelagert. Fig. 5 zeigt ein Foto des so behandelten Pflasters nach 2 Monaten. Es ist deutlich zu erkennen, dass die optische Inspektion kein Kristallwachstum an der Trennkante 18 erkennen lässt.

### Vergleichsbeispiel 2

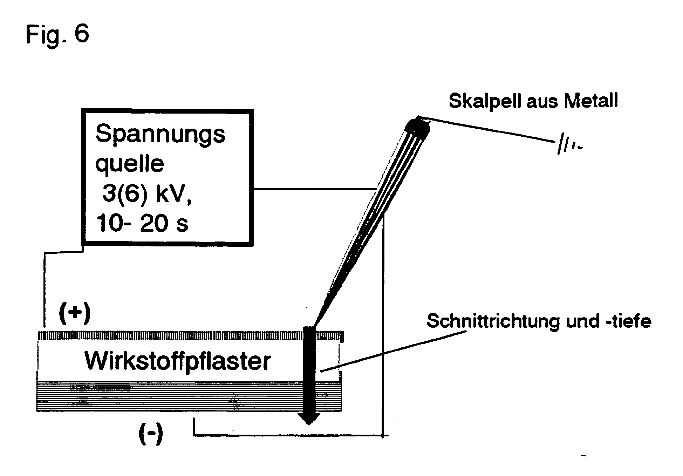

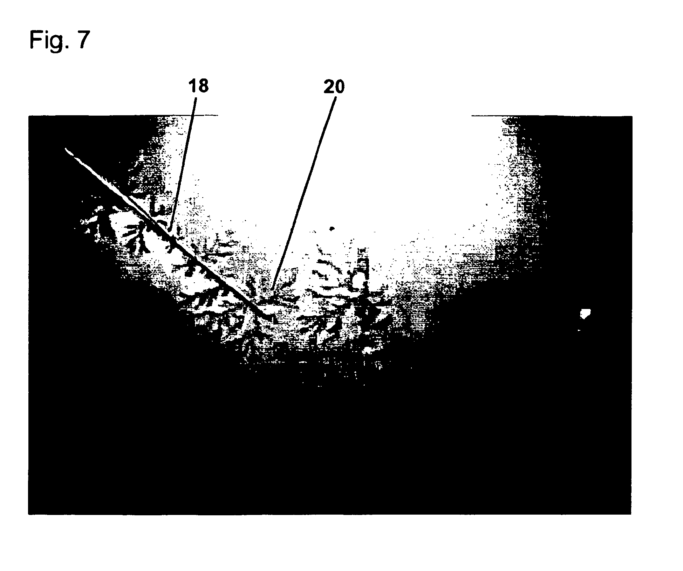

Die Folien eines kristallfreien Rotigotin-haltigen Pflasters wurden durch Anlegen einer Spannung von ca. 3 kV elektrisch aufgeladen (Fig. 6). Anschließend wurde das Pflaster an mehreren Stellen mittels eines elektrisch geerdeten Skalpells durchschnitten und dann bei Raumtemperatur für 1 Monat gelagert. Wie Fig. 7 zu entnehmen ist, die ein Foto dieser Trennkante (Schnittlinie) nach 1 Monat Lagerung bei Raumtemperatur zeigt, ist ein deutlich sichtbares Kristallwachstum 20 an der Trennkante 18 zu beobachten.

### Vergleichsbeispiel 3

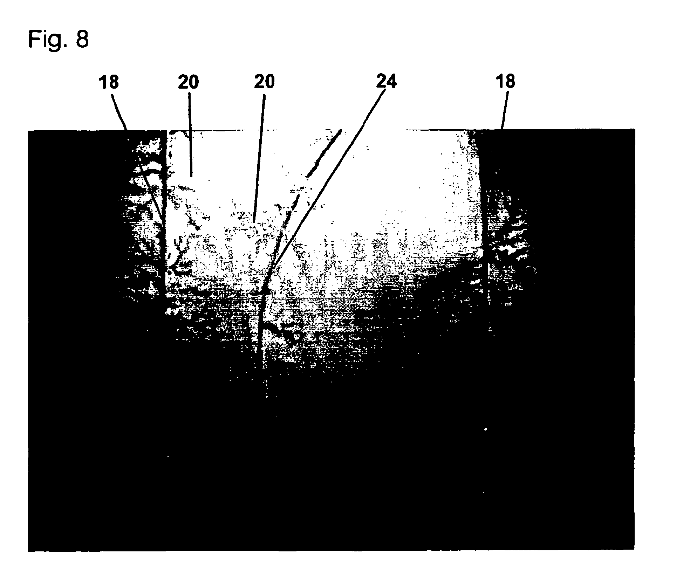

Ein Rotigotin-haltiges, kristallfreies, transdermales Pflaster, das bereits vor der behandlung mit einer Stanzlinie (z.B. S-Cut) 24 versehen war, wurde mit der Release-Liner-Seite nach unten auf einer Styroporplatte mittels Tesafilm befestigt. Die freie nicht auf der Styroporplatte aufliegende Trägerfolienseite des Pflasters wurde mit einem Naturhaarpinsel (Flachpinsel, ca. 2 cm breit) durch eindimensionale Reibung (über 10 sec) elektrostatisch bei normaler Luftfeuchte und Raumtemperatur aufgeladen. Anschließend wurde das Pflaster an mehreren Stellen wie in Beispiel 3 mittels eines elektrisch geerdeten Skalpells durchschnitten. Anschließend wurde das so behandelte Pflaster 1 Monat bei Raumtemperatur gelagert. Fig. 8 zeigt ein Foto, dass eine deutliche Kristallbildung 20 entlang der Schnittkanten 18 zu beobachten ist. Zusätzlich wurde eine Kristallisation 20 an der im Pflaster bereits vor der Behandlung vorhandenen Stanzlinie (S-Cut) 24 beobachtet.

## Patentansprüche

1. Transdermales Pflaster, umfassend
eine Release-Liner-Folie,
eine wirkstoffhaltige Polymerschicht mit einem amorphen Wirkstoff, und
eine Trägerschicht,
wobei die Wirkstoffschicht zwischen der Release-Liner-Folie und der Trägerschicht eingebracht ist, wobei
die Release-Liner-Folie und die Trägerschicht über eine Länge von mindestens 70% der umfangseitigen Trennkanten, thermisch und voneinander lösbar derart aneinander gebunden sind, dass die Wirkstoffschicht dadurch versiegelt ist, und wobei der Wirkstoff Rotigotin oder eines seiner pharmakologisch akzeptablen Salze ist.

2. Transdermales Pflaster nach Anspruch 1, wobei die Release-Liner-Folie und die Trägerschicht über die gesamte Länge der umfangseitigen Trennkanten aneinander gebunden sind.

3. Transdermales Pflaster nach einem der Ansprüche 1 und 2, wobei das thermisch aneinander Binden erfolgt durch Verfahren ausgewählt aus Schweißen, thermischem Verstemmen und/oder thermischem (Ver)Fügen.

4. Transdermales Pflaster nach einem der vorstehenden Ansprüche, wobei der Wirkstoff die freie Base von Rotigotin ist.

5. Transdermales Pflaster nach einem der vorstehenden Ansprüche, wobei die wirkstoffhaltige Polymerschicht aus einem silikonbasierten Kleber besteht, in dem der Wirkstoff in amorpher Form vorliegt.

6. Transdermales Pflaster nach einem der vorstehenden Ansprüche, bei dem zumindest die Release-Liner-Folie als Sollbruchkante einen geschwächten und/oder zumindest teilweise unterbrochenen Bereich aufweist, der als Öffnungshilfe für das Pflaster verwendbar ist.

7. Transdermales Pflaster nach einem der vorstehenden Ansprüche, bei dem die Release-Liner-Folie und die Trägerschicht aus demselben Material gefertigt sind.

8. Transdermales Pflaster nach einem der vorstehenden Ansprüche, wobei Rotigotin in der Wirkstoffschicht zu über 90 Mol% in Form der Wirkstoffbase und in 5-20 Gew% bezogen auf das Gewicht der Wirkstoffschicht vorliegt.

9. Transdermales Pflaster nach einem der vorstehenden Ansprüche zur Behandlung und/oder Prävention einer Erkrankung, ausgewählt aus Morbus Parkinson, Restless Leg Syndrom, Parkinson Plus, Depression, und Fibromyalgie.

10. Verfahren zur Herstellung eines transdermalen Pflasters, wobei zwischen einer Release-Liner-Folie und einer Trägerschicht eine Wirkstoffschicht mit Rotigotin und einem seiner pharmakologisch akzeptablen Salze als Wirkstoff angeordnet ist, wobei die Release-Liner-Folie und die Trägerschicht des Pflasters mit zumindest einer thermisch bewirkten umfangseitigen Trennkante, bevorzugt mit über ihre gesamte Länge thermisch bewirkten umfangseitigen Trennkanten versehen wird, und
wobei die Release-Liner-Folie und die Trägerschicht entlang der umfangseitigen Trennkanten über eine Länge von mindestens 70% der umfangseitigen Trennkanten, thermisch miteinander verbunden werden, so dass die Wirkstoffschicht durch die thermisch miteinander verbundenen Schichten versiegelt ist.

11. Verfahren nach Anspruch 10, wobei die Release-Liner-Folie und die Trägerschicht über die gesamte Länge der umfangseitigen Trennkanten thermisch miteinander verbunden werden.

12. Verfahren nach einem der Ansprüche 10 und 11, wobei die Release-Liner-Folie in einem Bereich einer Sollbruchkante, die als Öffnungshilfe für das Pflaster verwendbar ist, thermisch geschwächt und/oder zumindest teilweise unterbrochen wird.

13. Verfahren nach einem der Ansprüche 10 bis 12, wobei zumindest eine der thermischen Behandlungen oberhalb einer Schmelztemperatur der Wirkstoffkristalle von Rotigotin Form II durchgeführt wird.

14. Verfahren nach Anspruch 13, wobei die thermische Behandlung oberhalb einer Temperatur von 120°C durchgeführt wird.

15. Verfahren nach einem der Ansprüche 10 bis 14, wobei die Release-Liner-Folie und die Trägerschicht mit der Wirkstoffschicht versehen sind und wobei die Schichten mit der Wirkstoffschicht durch ein Paar beheizbare gegenläufige Walzen durchgeführt werden.

16. Verfahren nach einem der Ansprüche 10 bis 15, wobei unmittelbar bevor die Release-Liner-Folie und die Trägerschicht mit einer thermisch bewirkten Trennkante versehen werden, die Release-Liner-Folie und die Trägerschicht elektrisch entladen wird.

17. Verfahren nach einem der Ansprüche 10-16 umfassend die Verwendung eines Werkzeug, welches zwei gegenläufige, zumindest teilweise beheizbare Walzen umfasst, durch welche die Release-Liner-Folie und die Trägerschicht sowie die Wirkstoffschicht durchführbar sind.

18. Verfahren nach Anspruch 17, wobei eine erste Walze zur Vereinzelung des Pflasters eingerichtet ist und wobei eine zweite, zur ersten gegenläufige Walze zur Ausbildung eines geschwächten und/oder zumindest teilweise unterbrochenen Bereichs eingerichtet ist.

## Claims

1. Transdermal patch comprising
a release liner film,
an active ingredient-containing polymer layer containing an amorphous active ingredient, and
a support layer,
wherein the active ingredient layer is introduced between the release liner film and the support layer, wherein
the release liner film and the support layer are thermally and detachably bonded to one another over a length of at least 70% of the peripheral separation edges in such a way that the active ingredient layer is thereby sealed, and wherein the active ingredient is rotigotine or one of its pharmacologically acceptable salts.

2. Transdermal patch according to Claim 1, wherein the release liner film and the support layer are bonded to one another over the entire length of the peripheral separation edges.

3. Transdermal patch according to either of Claims 1 and 2, wherein bonding together in a thermal manner is achieved by methods selected from welding, thermal caulking and/or thermal joining.

4. Transdermal patch according to any of the preceding claims, wherein the active ingredient is the free base of rotigotine.

5. Transdermal patch according to any of the preceding claims, wherein the active ingredient-containing polymer layer consists of a silicone-based adhesive containing the active ingredient in amorphous form.

6. Transdermal patch according to any of the preceding claims, in which at least the release liner film has, as a predetermined breaking edge, a weakened and/or at least partly interrupted region usable as an opening aid for the patch.

7. Transdermal patch according to any of the preceding claims, in which the release liner film and the support layer are made from the same material.

8. Transdermal patch according to any of the preceding claims, wherein rotigotine is present in the active ingredient layer to an extent of over 90 mol% in the form of the active ingredient base and in 5-20% by weight based on the weight of the active ingredient layer.

9. Transdermal patch according to any of the preceding claims for treating and/or preventing a disorder selected from Parkinson's disease, restless legs syndrome, Parkinson-plus syndrome, depression and fibromyalgia.

10. Method for producing a transdermal patch, wherein an active ingredient layer containing rotigotine or one of its pharmacologically acceptable salts as active ingredient is arranged between a release liner film and a support layer, wherein the release liner film and the support layer of the patch is provided with at least one thermally effected peripheral separation edge, preferably with peripheral separation edges effected thermally over their entire length, and wherein the release liner film and the support layer are thermally bonded to one another along the peripheral separation edges over a length of at least 70% of the peripheral separation edges, and so the active ingredient layer is sealed by the layers thermally bonded to one another.

11. Method according to Claim 10, wherein the release liner film and the support layer are thermally bonded to one another over the entire length of the peripheral separation edges.

12. Method according to either of Claims 10 and 11, wherein the release liner film is thermally weakened and/or at least partly interrupted in a region of a predetermined breaking edge usable as an opening aid for the patch.

13. Method according to any of Claims 10 to 12, wherein at least one of the thermal treatments is carried out above a melting temperature of the active ingredient crystals of rotigotine form II.

14. Method according to Claim 13, wherein the thermal treatment is carried out above a temperature of 120°C.

15. Method according to any of Claims 10 to 14, wherein the release liner film and the support layer are provided with the active ingredient layer and wherein the layers containing the active ingredient layer are passed through a pair of heatable rollers working in opposite directions.

16. Method according to any of Claims 10 to 15, wherein, immediately before the release liner film and the support layer are provided with a thermally effected separation edge, the release liner film and the support layer are electrically discharged.

17. Method according to any of Claims 10-16, comprising the use of a tool comprising two at least partly heatable rollers working in opposite directions, through which it is possible to pass the release liner film and the support layer and also the active ingredient layer.

18. Method according to Claim 17, wherein a first roller is set up for isolation of the patch and wherein a second roller working in an opposite direction with respect to the first roller is set up for formation of a weakened and/or at least partly interrupted region.

## Revendications

1. Timbre transdermique, comprenant un film protecteur antiadhésif,
une couche de polymère contenant une substance active, comportant une substance active amorphe, et
une couche de support,
dans lequel la couche à substance active est introduite entre le film protecteur antiadhésif et la couche de support,
le film protecteur antiadhésif et la couche de support étant sur une longueur d'au moins 70 % des bords de séparation sur le pourtour liés l'un à l'autre thermiquement et en pouvant être détachés l'un de l'autre, de sorte que la couche à substance active est ainsi scellée, et la substance active étant la rotigotine ou un de ses sels pharmacologiquement acceptables.

2. Timbre transdermique selon la revendication 1, dans lequel le film protecteur antiadhésif et la couche de support sont liés l'un à l'autre sur toute la longueur des bords de séparation sur le pourtour.

3. Timbre transdermique selon l'une quelconque des revendications 1 et 2, dans lequel la liaison l'un à l'autre thermiquement s'effectue par des procédés choisis parmi le soudage, le matage thermique et/ou le jointoiement (aboutement) thermique.

4. Timbre transdermique selon l'une quelconque des revendications précédentes, dans lequel la substance active est la base libre de la rotigotine.

5. Timbre transdermique selon l'une quelconque des revendications précédentes, dans lequel la couche de polymère contenant une substance active consiste en une colle à base de silicone, dans laquelle la substance active se trouve sous forme amorphe.

6. Timbre transdermique selon l'une quelconque des revendications précédentes, dans lequel au moins le film protecteur antiadhésif présente en tant que bord prévu pour la rupture une zone affaiblie et/ou au moins en partie interrompue, qui est utilisable comme aide à l'ouverture pour le timbre.

7. Timbre transdermique selon l'une quelconque des revendications précédentes, dans lequel le film protecteur antiadhésif et la couche de support sont produits à partir du même matériau.

8. Timbre transdermique selon l'une quelconque des revendications précédentes, dans lequel la rotigotine dans la couche à substance active est présente à raison de plus de 90 % en moles sous forme de la base de substance active et en 5-20 % en poids par rapport au poids de la couche à substance active.

9. Timbre transdermique selon l'une quelconque des revendications précédentes, destiné au traitement et/ou à la prévention d'une maladie, choisie parmi la maladie de Parkinson, le syndrome des jambes sans repos, le Parkinson plus, la dépression et la fibromyalgie.

10. Procédé pour la fabrication d'un timbre transdermique, dans lequel entre un film protecteur antiadhésif et une couche de support est disposée en tant que substance active une couche à substance active comportant de la rotigotine ou un de ses sels pharmacologiquement acceptables, le film protecteur antiadhésif et la couche de support du timbre étant dotés d'au moins un bord de séparation sur le pourtour, engendré thermiquement, de préférence de bords de séparation sur le pourtour, engendrés thermiquement sur toute leur longueur, et le film protecteur antiadhésif et la couche de support étant liés l'un à l'autre thermiquement le long des bords de séparation sur le pourtour sur une longueur d'au moins 70 % des bords de séparation sur le pourtour, de sorte que la couche à substance active est scellée par les couches liées thermiquement entre elles.

11. Procédé selon la revendication 10, dans lequel le film protecteur antiadhésif et la couche de support sont liés thermiquement l'un à l'autre sur toute la longueur des bords de séparation sur le pourtour.

12. Procédé selon l'une quelconque des revendications 10 et 11, dans lequel le film protecteur antiadhésif est affaibli et/ou au moins en partie interrompu dans une zone d'un bord prévu pour la rupture, qui est utilisable comme aide à l'ouverture pour le timbre.

13. Procédé selon l'une quelconque des revendications 10 à 12, dans lequel au moins l'un des traitements thermiques est effectué au-dessus d'une température de fusion des cristaux de substance active de la forme II de la rotigotine.

14. Procédé selon la revendication 13, dans lequel on effectue le traitement thermique au-dessus d'une température de 120 °C.

15. Procédé selon l'une quelconque des revendications 10 à 14, dans lequel le film protecteur antiadhésif et la couche de support sont munis de la couche à substance active et on fait passer les couches comportant la couche à substances active par une paire de cylindres contrarotatifs pouvant être chauffés.

16. Procédé selon l'une quelconque des revendications 10 à 15, dans lequel immédiatement avant que le film protecteur antiadhésif et la couche de support soient dotés d'un bord de séparation engendré thermiquement, on décharge électriquement le film protecteur antiadhésif et la couche de support.

17. Procédé selon l'une quelconque des revendications 10 à 16, comprenant l'utilisation d'un outil, qui comprend deux cylindres contrarotatifs pouvant être chauffés au moins en partie, par lesquels peuvent être passés le film protecteur antiadhésif et la couche de support ainsi que la couche à substance active.

18. Procédé selon la revendication 17, dans lequel un premier cylindre est destiné à l'isolement du timbre et dans lequel un deuxième cylindre, contrarotatif par rapport au premier, est destiné à la formation d'une zone affaiblie et/ou au moins en partie interrompue.
